# EUROPEAN PATENT APPLICATION

(11) **EP 4 272 807 A1**
(43) Date of publication of application: **08.11.2023**
(21) Application number: 21913981.3
(22) Date of filing: 20.12.2021
(51) Int. Cl.: A61N 1/362

(54) **CARDIAC PACING SYSTEM**

(30) Priority: 30.12.2020 CN 202011613881
(71) Applicant: MicroPort Soaring CRM (Shanghai) Co., Ltd., Shanghai 201203 (CN)
(72) Inventor: LIU, Gang, Shanghai 201203 (CN)
(74) Representative: Lavoix
(86) International application number: PCT/CN2021/139464
(87) International publication number: WO 2022/143240

(57) **Abstract**

A cardiac pacing system is disclosed, an energy recovery module is connected to a second on-off switching unit and then between a node between a first on-off switching unit and a DC blocking capacitor and electrical ground, and an energy storage module is connected to the energy recovery module. When the first on-off switching unit is closed and the second on-off switching unit is open, a battery normally delivers pacing pulses to myocardial tissue. When the first on-off switching unit is open and the second on-off switching unit is closed, the energy recovery module recovers energy from the DC blocking capacitor and stores it in the energy storage module. The recovered energy can be used for subsequent pacing or normal operation of other modules in the cardiac pacing system, reducing a waste of energy while not adding additional power consumption. Such energy savings of the battery in the cardiac pacing system can extend a service life of the cardiac pacing system.

## Description

### TECHNICAL FIELD

The present invention relates to the field of implantable medical devices and, in particular, to a cardiac pacing system.

### BACKGROUND

Cardiac pacemaker systems have advanced tremendously since their advent in the 1950s in terms of both indications and functions. In addition to pacing, cardiac pacing systems have been used also in various diagnostic techniques relating to arrhythmia.

Well-known cardiac pacing systems include a cardiac pacemaker and electrode leads, which are both surgically implanted in a human body. The pacemaker is powered by a battery to deliver, as needed, electrical pulses (i.e., pacing pulses) with tiny amounts of energy from the battery to myocardial tissue. After passing through the electrode leads and a DC blocking capacitor, the pacing pulses reach the myocardial tissue (the heart disallows the passage of DC currents therethrough), applying stimuli to the myocardial tissue that electrodes of the electrode leads are brought into contact with, which cause the heart to beat and contract and thereby provide a therapeutic effect against certain heart dysfunctions caused by arrhythmia.

However, in such conventional cardiac pacing systems, after each pacing pulse is delivered, a certain amount of charge remains on the DC blocking capacitor depending on the pacing pulse's amplitude and pulse width and impedance of the patient's myocardial tissue. For this reason, the delivery of each pacing pulse is followed by discharge of the DC blocking capacitor in order to ensure effective cardiac pacing of the next pulse. Conventionally, this is accomplished by direct discharge of the remaining charge on the DC blocking capacitor to electrical ground through a switch. This approach suffers from a waste of energy of the battery, greatly affecting the service life of the cardiac pacing system.

### SUMMARY OF THE INVENTION

It is an objective of the present invention to overcome the problem associated with conventional cardiac pacing systems of a reduced service life due to a waste of battery energy by presenting a novel cardiac pacing system.

To this end, the present invention provides a cardiac pacing system including a first on-off switching unit, a DC blocking capacitor and an energy recovery circuit, which are connected in sequence between a battery and electrical ground, the DC blocking capacitor connected to myocardial tissue which is in turn connected to electrical ground, the energy recovery circuit including an energy recovery module, a second on-off switching unit and an energy storage module,
wherein the energy recovery module is connected to the second on-off switching unit and then between a node between the first on-off switching unit and the DC blocking capacitor and electrical ground, and the energy storage module is connected to the energy recovery module; and
when the first on-off switching unit is closed and the second on-off switching unit is open, the battery delivers pacing pulses to the myocardial tissue, and when the first on-off switching unit is open and the second on-off switching unit is closed, the energy recovery module recovers energy from the DC blocking capacitor and stores it in the energy storage module.

Optionally, the energy recovery module may include a low-voltage oscillation unit and a low-voltage charge pump unit, wherein a power supply terminal of the low-voltage oscillation unit and a power supply terminal of the low-voltage charge pump unit are both configured to input voltage on the node; a ground terminal of the low-voltage oscillation unit and a ground terminal of the low-voltage charge pump unit are both connected to electrical ground;
an output terminal of the low-voltage oscillation unit is connected to an input terminal of the low-voltage charge pump unit; the low-voltage oscillation unit is configured to input two non-overlapping clock signals to the low-voltage charge pump unit; an output terminal of the low-voltage charge pump unit is connected to the energy storage module; and the low-voltage charge pump unit is configured to output a voltage to the energy storage module under the drive of the clock signals.

Optionally, the low-voltage oscillation unit may include a low-voltage oscillation circuit and a low-voltage buffer circuit, wherein a power supply terminal of the low-voltage oscillation circuit and a power supply terminal of the low-voltage buffer circuit together serve as the power supply terminal of the low-voltage oscillation unit, wherein a ground terminal of the low-voltage oscillation circuit and a ground terminal of the low-voltage buffer circuit together serve as the ground terminal of the low-voltage oscillation unit; and
an output terminal of the low-voltage oscillation circuit is connected to an input terminal of the low-voltage buffer circuit; the low-voltage oscillation circuit is configured to output two non-overlapping initial clock signals to the low-voltage buffer circuit; an output terminal of the low-voltage buffer circuit is connected to the input terminal of the low-voltage charge pump unit; and the low-voltage buffer circuit is configured to augment driving power of the two initial clock signals and then output the two clock signals to the low-voltage charge pump unit.

Optionally, the low-voltage oscillation circuit may include m stages first inverters, where m is an odd number greater than or equal to 3,
wherein power supply terminals of the m stages first inverters are connected and together serve as the power supply terminal of the low-voltage oscillation circuit; ground terminals of the m stages first inverters are connected and together serve as the ground terminal of the low-voltage oscillation circuit; an output terminal of an m-th stage first inverter is connected to an input terminal of a first-stage first inverter; an output terminal of an i-th stage first inverter is connected to an input terminal of an (i+1)-th stage o first inverter, where 1<i≤m-1; and
the two initial clock signals are output from the output terminals respectively of any odd number-stage of the first inverters and any even number-stage of the first inverters.

Optionally, each of the first inverter stages may include a first PMOS transistor and a first NMOS transistor,
the first PMOS transistor having a source serving as the power supply terminal of the first inverter, the first PMOS transistor having a gate connected to a gate of the first NMOS transistor and serving as the input terminal of the first inverter, the first PMOS transistor having a drain connected to a drain of the first NMOS transistor and serving as the output terminal of the first inverter, the first NMOS transistor having a source serving as the ground terminal of the first inverter, the first NMOS transistor having a substrate connected to a substrate of the first PMOS transistor and serving as a feedback terminal of the first inverter,
wherein the feedback terminal of the m-th stage first inverter is connected to the output terminal of the first-stage first inverter, and the feedback terminal of a j-th stage first inverter is connected to the output terminal of a (j+1)-th stage first inverter, where 1≤j≤m-1.

Optionally, the low-voltage buffer circuit may include two buffers each including n stages second inverters, where n≥2,
wherein power supply terminals of the n stage second inverters are connected and together serve as the power supply terminal of the low-voltage buffer circuit; ground terminals of the n stages second inverters are connected and together serve as the ground terminal of the low-voltage buffer circuit; an output terminal of an r-th stage second inverter is connected to an input terminal of an (r+1)-th stage second inverter; an input terminal of a first-stage second inverter and an output terminal of an n-th stage second inverter serve as an input terminal and an output terminal of the buffer, where 1≤r≤n-1; and
the two initial clock signals are input to the respective input terminals of the two buffers, and the clock signals are output from the respective output terminals of the two buffers.

Optionally, each of the second inverter stages may include a second PMOS transistor and a second NMOS transistor,
the second PMOS transistor having a source serving as the power supply terminal of the second inverter, the second PMOS transistor having a gate connected to a gate of the second NMOS transistor and serving as the input terminal of the second inverter, the second PMOS transistor having a drain connected to a drain of the second NMOS transistor and serving as the output terminal of the second inverter, the second NMOS transistor having a source serving as the ground terminal of the second inverter, the second NMOS transistor having a substrate connected to a substrate of the second PMOS transistor and serving as a feedback terminal of the second inverter,
wherein when the initial clock signal input to the buffer is output from an odd number-stage of the first inverters, the feedback terminal of each odd number-stage of the second inverters in the buffer is connected to the feedback terminal of any even number-stage of the first inverters, and the feedback terminal of each even number-stage of the second inverters in the buffer is connected to the feedback terminal of any odd number-stage of the first inverters; and
when the initial clock signal input to the buffer is output from an even number-stage of the first inverters, the feedback terminal of each odd number-stage of the second inverters in the buffer is connected to the feedback terminal of any odd number-stage of the first inverters, and the feedback terminal of each even number-stage of the second inverters in the buffer is connected to the feedback terminal of any even number-stage of the first inverters.

Optionally, the low-voltage charge pump unit may include q stages charge pump elements, where q ≥2,
each of the charge pump elements including a third NMOS transistor and a coupling capacitor, the third NMOS transistor having a gate, a drain and a substrate, which are connected and together serve as a power supply terminal of the charge pump element, the third NMOS transistor having a source connected to one end of the coupling capacitor and serving as an output terminal of the charge pump element, the other end of the coupling capacitor serving as an input terminal of the charge pump element,
wherein the output terminal of an s-th stage of the charge pump elements is connected to the power supply terminal of an (s+1)-th stage of the charge pump elements, and the power supply terminal of a first stage of the charge pump elements and the output terminal of a q-th stage of the charge pump elements serve as the power supply terminal and the output terminal of the low-voltage charge pump unit, respectively, where 1≤s≤q-1; and
the input terminals of odd number-stages of the charge pump elements are connected and together serve as one input terminal of the low-voltage charge pump unit, the input terminal of each odd number-stage of the charge pump elements is configured for input of one of the clock signals, the input terminals of even number-stages of the charge pump elements are connected and together serve as another input terminal of the low-voltage charge pump unit, and the input terminal of each even number-stage of the charge pump elements is configured for input of the other one of the clock signals.

Optionally, the energy recovery circuit may further include a third on-off switching unit, one end of the third on-off switching unit is connected between the energy recovery module and the second on-off switching unit, and the other end of the third on-off switching unit is connected to electrical ground.

Optionally, the energy recovery circuit may further include a third on-off switching unit, one end of the third on-off switching unit is connected between the node and the second on-off switching unit, and the other end of the third on-off switching unit is connected to electrical ground.

Optionally, during the delivery of the pacing pulses by the battery to the myocardial tissue, the recovery of energy by the energy recovery module from the DC blocking capacitor and the storage of the energy in the energy storage module, the third on-off switching unit may be open.

Optionally, after the energy recovery module recovers energy from the DC blocking capacitor for a predetermined period of time, both the third on-off switching unit and the second on-off switching unit may be closed, thereby releasing remaining energy in the DC blocking capacitor.

Optionally, after the energy recovery module recovers energy from the DC blocking capacitor for a predetermined period of time, the third on-off switching unit may be closed and the second on-off switching unit may be open, thereby releasing remaining energy in the DC blocking capacitor.

In the cardiac pacing systems provided in the present invention, the second on-off switching unit of the energy recovery module is connected between the node between the first on-off switching unit and the DC blocking capacitor and electrical ground, and the energy storage module is connected to the energy recovery module. When the first on-off switching unit is closed and the second on-off switching unit is open, the battery can normally deliver pacing pulses to myocardial tissue. When the first on-off switching unit is open and the second on-off switching unit is closed, the energy recovery module recovers energy from the DC blocking capacitor and stores it in the energy storage module. The recovered energy can be used for subsequent pacing or normal operation of other modules in the cardiac pacing system, reducing a waste of energy while not adding additional power consumption. Such energy savings of the battery in the cardiac pacing system can extend a service life of the cardiac pacing system.

### BRIEF DESCRIPTION OF THE DRAWINGS

Fig. 1a shows a circuit diagram of a cardiac pacing system according to a first embodiment of the present invention.
Fig. 1b schematically illustrates control signals for a pacing switch and an energy recovery switch in the first embodiment of the present invention.
Fig. 2 is a diagram showing an equivalent circuit of an energy recovery module in the first embodiment of the present invention.
Fig. 3 is a diagram showing an equivalent circuit of a low-voltage oscillation unit in the first embodiment of the present invention.
Fig. 4 is a circuit diagram of a low-voltage oscillation circuit and a low-voltage buffer circuit in the first embodiment of the present invention.
Fig. 5 is a circuit diagram of a low-voltage charge pump unit in the first embodiment of the present invention.
Fig. 6 is a diagram showing simulation results of an energy recovery circuit in the first embodiment of the present invention.
Fig. 7a is a circuit diagram of a cardiac pacing system according to a second embodiment of the present invention;
Fig. 7b schematically illustrates control signals for a pacing switch, an energy recovery switch and a discharge switch in the second embodiment of the present invention.
Fig. 8a is a circuit diagram of a cardiac pacing system according to a third embodiment of the present invention;
Fig. 8b schematically illustrates control signals for a pacing switch, an energy recovery switch and a discharge switch in the third embodiment of the present invention.

In these figures:
K1-pacing switch; K2-energy recovery switch; K3-discharge switch; P-node; C1-direct-current (DC) blocking capacitor; C2-coupling capacitor; W-myocardial tissue; CLK-first clock signal; CLKN-second clock signal; CLK_F-first initial clock signal; CLKN_F-second initial clock signal; Q11-first PMOS transistor; Q12-first NMOS transistor; Q21-second PMOS transistor; Q22-second NMOS transistor; Q31-third NMOS transistor; Vin-voltage;
10-battery; 20-energy recovery module; 21-low-voltage oscillation unit; 211-low-voltage oscillation circuit; 212-low-voltage buffer circuit; 212a-first buffer; 212b-second buffer; 22-low-voltage charge pump unit; 30-energy storage module.

### DETAILED DESCRIPTION

Specific embodiments of the present invention will be described in greater detail below with reference to the accompanying drawings. From the following description, advantages and features of the present invention will become more apparent. Note that the figures are provided in a very simplified form not necessarily drawn to exact scale and for the only purpose of facilitating easy and clear description of the embodiments.

### Embodiment 1

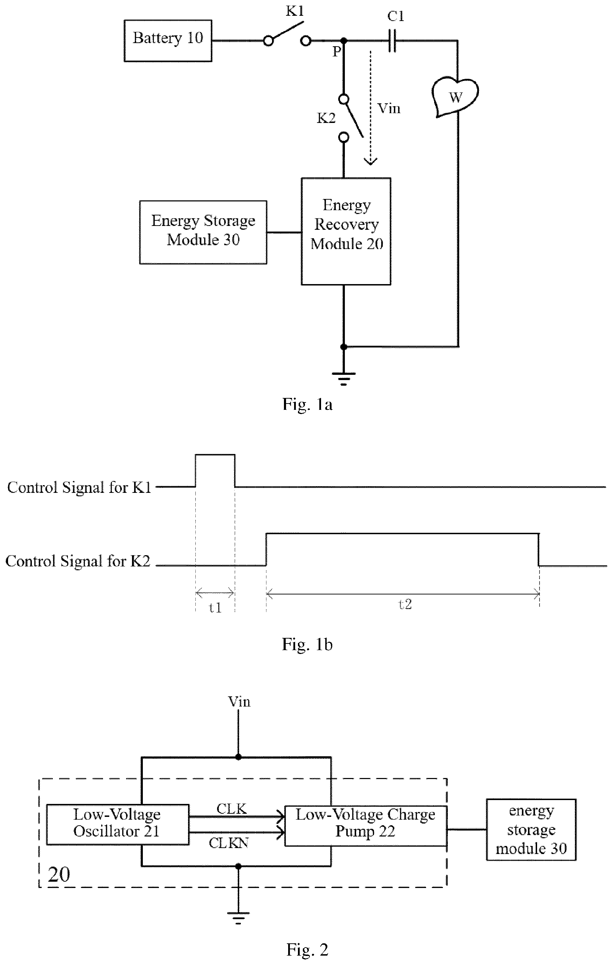

Fig. 1a shows a circuit diagram of a cardiac pacing system according to a first embodiment. As shown in Fig. 1a, the cardiac pacing system according to this embodiment includes a first on-off switching unit, a DC blocking capacitor C1 and an energy recovery circuit, which are connected in sequence between a battery 10 and electrical ground. The DC blocking capacitor C1 is connected to myocardial tissue W, the myocardial tissue W is connected to electrical ground. The energy recovery circuit includes an energy recovery module 20, a second on-off switching unit and an energy storage module 30. The energy recovery module 20 is connected to the second on-off switching unit and then between a node P between the first on-off switching unit and the second on-off switching unit and electrical ground, and the energy storage module 30 is connected to the energy recovery module 20.

In this embodiment, the term "electrical ground" refers to operating ground (neutral ground) required for operation of the circuit modules.

In this embodiment, both the first on-off switching unit and the second on-off switching unit are implemented as switches. Specifically, the first on-off switching unit is implemented as a pacing switch K1, and the second on-off switching unit as an energy recovery switch K2. It would be appreciated that the first on-off switching unit and the second on-off switching unit are not limited to being implemented as switches, as each of them may be alternatively implemented as any other device or combination of devices capable of switching on and off a circuit. For example, each of the first on-off switching unit and the second on-off switching unit may be alternatively implemented as a relay or a combination of multiple switches, but further description thereof is deemed unnecessary and is omitted.

Specifically, as shown in Fig. 1a, in this embodiment, the battery 10 is connected to one end of the pacing switch K1, and the other end of the pacing switch K1 is connected to the node P. The node P is connected to one end of the energy recovery switch K2, and the other end of the energy recovery switch K2 is connected to a power supply terminal of the energy recovery module 20. A ground terminal of the energy recovery module 20 is connected to electrical ground. The node P is also connected to one end of the DC blocking capacitor C1, and the other end of the DC blocking capacitor C1 is connected to one location of the myocardial tissue W. Another location of the myocardial tissue W is connected to electrical ground.

Fig. 1b schematically illustrates control signals for the pacing switch K1 and the energy recovery switch K2 in this embodiment. As shown in Fig. 1b, in a time interval t1, the pacing switch K1 is closed and the energy recovery switch K2 is open. As a result, the energy recovery module 20 is turned off, and pacing pulses are output from the battery 10. After propagating through the DC blocking capacitor C1 which filters out a DC component thereof, the pacing pulses are transmitted to and stimulate the myocardial tissue W, causing the heart to beat and contract. With continued reference to Fig. 1b, in a time interval t2, the pacing switch K1 is open and the energy recovery switch K2 is closed, turning off the battery 10. Charge on the DC blocking capacitor C1 flows into the energy recovery module 20 (manifested as a voltage Vin at the node P being applied to the energy recovery module 20). The energy recovery module 20 receives the charge and utilizes it to charge the energy storage module 30. As such, energy can be recovered by the energy recovery module 20 from the DC blocking capacitor C1 and stored in the energy storage module 30.

The energy storage module 30 may be a module or device capable of storing electrical charges, such as a capacitor or the like. Energy recovered by the energy storage module 30 may be used to charge the battery 10 (therefore, in this case, the battery 10 is a rechargeable battery), for example, through an additional charge/discharge circuit, increasing the power of the battery 10 for subsequent pacing. The energy storage module 30 may also be connected to other modules (e.g., a sensing module or a sampling module, etc.) in the cardiac pacing system in order to provide these modules with energy required for their normal operation. Thus, with the present invention, a waste of energy from the battery 10 can be reduced while not adding additional power consumption. Such energy savings of the battery 10 can extend a service life of the cardiac pacing system.

Fig. 2 is a diagram showing an equivalent circuit of the energy recovery module 20 in this embodiment. As shown in Figs. 1a and 2, the energy recovery module 20 includes a low-voltage oscillation unit 21 and a low-voltage charge pump unit 22. A power supply terminal of the low-voltage oscillation unit 21 and a power supply terminal of the low-voltage charge pump unit 22 are both connected to the other end of the energy recovery switch K2. In this way, the voltage Vin can be input to the low-voltage oscillation unit 21 and the low-voltage charge pump unit 22 through their power supply terminals. Ground terminals of the low-voltage oscillation unit 21 and ground terminals of the low-voltage charge pump unit 22 are both connected to electrical ground.

With continued reference to Fig. 2, an output terminal of the low-voltage oscillation unit 21 is connected to input terminals of the low-voltage charge pump unit 22, thereby enabling two non-overlapping clock signals to be input to the low-voltage charge pump unit 22. For ease of distinction, the two clock signals are respectively referred to as a first clock signal CLK and a second clock signal CLKN. Moreover, an output terminal of the low-voltage charge pump unit 22 is connected to the energy storage module 30, thereby enabling a voltage to be output to the energy storage module 30 under the drive of the first clock signal CLK and the second clock signal CLKN. As such, the low-voltage charge pump unit 22 functions as if it was driven by the low-voltage oscillation unit 21 to charge the energy storage module 30. Thus, charge on the DC blocking capacitor C1 can be transferred to and stored on the energy storage module 30 without adding additional power consumption.

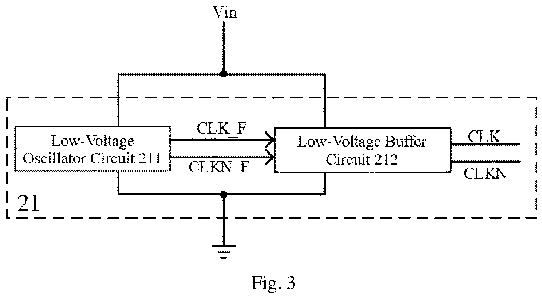

Fig. 3 is a diagram showing an equivalent circuit of the low-voltage oscillation unit 21 in this embodiment. As shown in Figs. 2 and 3, the low-voltage oscillation unit 21 includes a low-voltage oscillation circuit 211 and a low-voltage buffer circuit 212. A power supply terminal of the low-voltage oscillation circuit 211 and a power supply terminal of the low-voltage buffer circuit 212 are both connected to the other end of the energy recovery switch K2. In this way, the power supply terminals of the low-voltage oscillation circuit 211 and the low-voltage buffer circuit 212 can both act as the power supply terminal of the low-voltage oscillation unit 21 for input of the voltage Vin, and ground terminals of the low-voltage oscillation circuit 211 and the low-voltage buffer circuit 212 can both serve as the ground terminal of the low-voltage oscillation unit 21 for connection with electrical ground.

With continued reference to Fig. 3, an output terminal of the low-voltage oscillation circuit 211 is connected to an input terminal of the low-voltage buffer circuit 212, thereby enabling two non-overlapping initial clock signals to be output to the low-voltage buffer circuit 212. For ease of distinction, the two initial clock signals are respectively referred to as a first initial clock signal CLK_F and a second initial clock signal CLKN_F. An output terminal of the low-voltage buffer circuit 212 is connected to the input terminals of the low-voltage charge pump unit 22, thereby augmenting driving power of the first initial clock signal CLK_F and the second initial clock signal CLKN_F and thus converting them to the first clock signal CLK and the second clock signal CLKN to be output to the low-voltage charge pump unit 22, respectively.

Since the voltage Vin is typically low, driving power of the first initial clock signal CLK_F and the second initial clock signal CLKN_F output from the low-voltage oscillation circuit 211 is weak and typically insufficient to drive the low-voltage charge pump unit 22. In this embodiment, the low-voltage buffer circuit 212 is actually intended to increase the driving power of the clock signals and thus enable the first clock signal CLK and the second clock signal CLKN to more easily drive the low-voltage charge pump unit 22. As an optional alternative, in order to reduce cost, the low-voltage buffer circuit 212 may be omitted, and the first initial clock signal CLK_F and the second initial clock signal CLKN_F output from the low-voltage oscillation circuit 211 may be directly input to the low-voltage charge pump unit 22 as the clock signals.

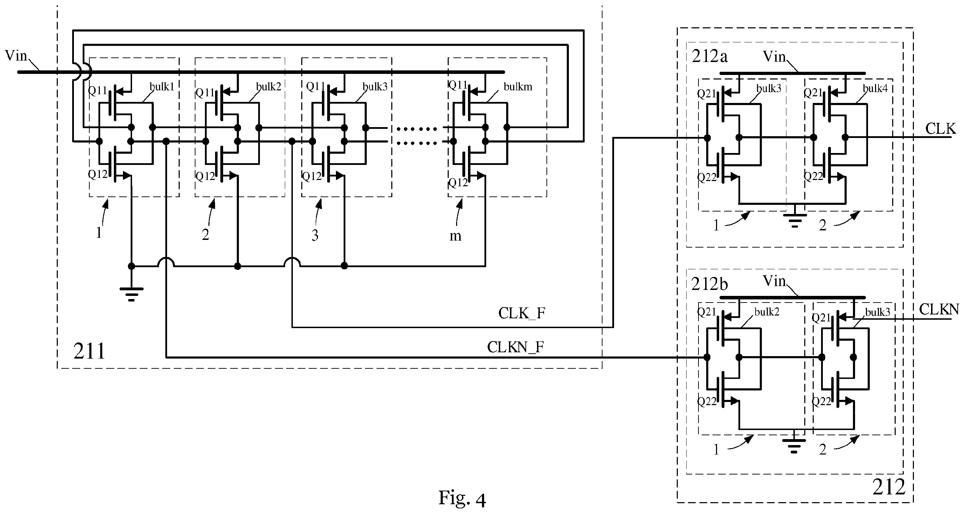

Fig. 4 is a circuit diagram of the low-voltage oscillation circuit 211 and the low-voltage buffer circuit 212 in this embodiment. As shown in Fig. 4, the low-voltage oscillation circuit 211 includes m-stage first inverters, such as first, second, third, ..., m-stage first inverters as shown in Fig. 4, where m is an odd number greater than or equal to 3.

With continued reference to Fig. 4, power supply terminals of the m-stage first inverters are connected and together serve as the power supply terminal of the low-voltage oscillation circuit 211. In this way, the voltage Vin can be input to all the m-stage first inverters through their respective power supply terminals. Ground terminals of the m-stage first inverters are connected and together serve as the ground terminal of the low-voltage oscillation circuit 211 for connection with electrical ground. Moreover, output terminals of the m-stage first inverters are connected to an input terminal of the first-stage first inverter, and output terminals of the i-stage first inverters are connected to input terminals of the (i+1)-stage first inverters, where 1<i≤m-1. As such, the m-stage first inverters are connected in series into a loop, in this way, the low-voltage oscillation circuit 211 is able to provide a voltage in both directions and it can be robustly powered through the connecting in series into a loop. Upon failure of any portion of the loop, this portion may be disconnected from the loop with a switch, without affecting the powering of the rest of the loop.

The first initial clock signal CLK_F and the second initial clock signal CLKN_F are output from the output terminals of any odd number-stage first inverter and any even number-stage first inverter, respectively. In this embodiment, the first initial clock signal CLK_F is output from the output terminal of the first-stage first inverter, and the second initial clock signal CLKN_F is output from the output terminal of the second-stage first inverter. However, this should not be construed as limiting the present invention in any sense.

With continued reference to Fig. 4, in this embodiment, each first inverter includes a first PMOS transistor Q11 and a first NMOS transistor Q12. A source of the first PMOS transistor Q11 serves as the power supply terminal of the first inverter, and a gate of the first PMOS transistor Q11 is coupled to a gate of the first NMOS transistor Q12 and serves as the input terminal of the first inverter. Moreover, a drain of the first PMOS transistor Q11 is coupled to a drain of the first NMOS transistor Q12 and serves as the output terminal of the first inverter. A source of the first NMOS transistor Q12 serves as the ground terminal of the first inverter, and a substrate thereof is connected to a substrate of the first PMOS transistor Q11 and serves as a feedback terminal of the first inverter.

Moreover, in this embodiment, the feedback terminal of the m-stage first inverter is connected to the output terminal of the first-stage first inverter, and the feedback terminal of the j-stage first inverter is coupled to the output terminal of the (j+1)-stage first inverter, where 1≤j≤m-1. In other words, the feedback terminal of a preceding-stage first inverter is connected to the output terminal of the succeeding-stage first inverter. Since levels output from the output terminals of the two stage first inverters have opposite polarities, when the succeeding-stage first inverter outputs a high level, substrate voltages of the first NMOS transistor Q12 and the first PMOS transistor Q11 in the preceding-stage first inverter will be raised. As a result, a threshold voltage of the first NMOS transistor Q12 in the preceding-stage first inverter will be reduced, and a threshold voltage of the first PMOS transistor Q11 will be increased, facilitating output of a low level by the preceding-stage first inverter from the output terminal thereof. Likewise, a low level output from the output terminal of the succeeding-stage first inverter facilitates output of a high level from the preceding-stage first inverter. In this way, the voltage Vin at which the low-voltage oscillation circuit 211 in this embodiment operates may be below the threshold voltage of the first NMOS transistors Q12 or the first PMOS transistors Q11.

With continued reference to Fig. 4, the low-voltage buffer circuit 212 includes two buffers: a first buffer 212a and a second buffer 212b. The first initial clock signal CLK_F and the second initial clock signal CLKN_F are input to the first buffer 212a and the second buffer 212b, respectively. The first buffer 212a and the second buffer 212b augment the driving power of the first initial clock signal CLK_F and the second initial clock signal CLKN_F, respectively, and output the first clock signal CLK and the second clock signal CLKN, correspondingly. In this way, the first clock signal CLK and the second clock signal CLKN can more easily drive the low-voltage charge pump unit 22.

Further, each of the first buffer 212a and the second buffer 212b includes n-stage second inverters, where n≥2. Power supply terminals of the n-stage second inverters are connected and together serve as the power supply terminal of the low-voltage buffer circuit 212. In this way, the voltage Vin can be input to all the n-stage second inverters through their respective power supply terminals. Ground terminals of the n-stage second inverters are connected and together serve as the ground terminal of the low-voltage buffer circuit 212 for connection with electrical ground. Further, an output terminal of the r-stage second inverter is coupled to an input terminal of the (r+1)-stage second inverter, where 1≤r≤n-1. The input terminal of the first-stage second inverter serves as an input terminal of the first buffer 212a or the second buffer 212b for input of the first initial clock signal CLK_F or the second initial clock signal CLKN_F. The output terminal of the n-stage second inverter serves as an output terminal of the first buffer 212a or the second buffer 212b for output of the first clock signal CLK or the second clock signal CLKN. In this way, the n-stage second inverters are connected in series.

With continued reference to Fig. 4, each second inverter includes a second PMOS transistor Q21 and a second NMOS transistor Q22. A source of the second PMOS transistor Q21 serves as a power supply terminal of the second inverter, a gate thereof is coupled to a gate of the second NMOS transistor Q22 and serves as the input terminal of the second inverter. Moreover, a drain of the second PMOS transistor Q21 is coupled to a drain of the second NMOS transistor Q22 and serves as the output terminal of the second inverter. A source of the second NMOS transistor Q22 serves as the ground terminal of the second inverter, and a substrate thereof is connected to a substrate of the second PMOS transistor Q21 and serves as a feedback terminal of the second inverter.

Further, since the first initial clock signal CLK_F is output from an even number-stage first inverter, the feedback terminal of each odd number-stage second inverter in the first buffer 212a is coupled to the feedback terminal of any odd number-stage first inverter, and the feedback terminal of each even number-stage second inverter in the first buffer 212a is coupled to the feedback terminal of any even number-stage first inverter. Since the second initial clock signal CLKN_F is output from an odd number-stage first inverter, the feedback terminal of each odd number-stage second inverter in the second buffer 212b is coupled to the feedback terminal of any even number-stage first inverter, and the feedback terminal of each even number-stage second inverter in the second buffer 212b is coupled to the feedback terminal of any odd number-stage first inverter. Likewise, this wiring scheme enables the voltage Vin at which the low-voltage buffer circuit 212 operates to be below the threshold voltage of the second NMOS transistors Q22 or the second PMOS transistors Q21. For ease of understanding, a more detailed description is set forth below with reference to Fig. 4.

As shown in Fig. 4, a desirable driving power augmentation effect can be obtained with only two stage second inverters (i.e., when n=2). Accordingly, in this embodiment, each of the first buffer 212a and the second buffer 212b includes two stage second inverters referred to respectively as a first-stage second inverter and a second-stage second inverter. The feedback terminals of the first, second, third, ..., m stage first inverters are denoted as bulk1, bulk2, bulk3,..., bulkm. In the first buffer 212a, the feedback terminal of the first-stage second inverter is connected to the feedback terminal bulk3 of the third-stage first inverter (i.e., the feedback terminal of an odd number-stage first inverter), and the feedback terminal of the second-stage second inverter is coupled to the feedback terminal bulk4 of the fourth-stage first inverter (i.e., the feedback terminal of an even number-stage first inverter). In the second buffer 212b, the feedback terminal of the first-stage second inverter is coupled to the feedback terminal bulk2 of the second-stage first inverter (i.e., the feedback terminal of an even number-stage first inverter), and the feedback terminal of the second-stage second inverter is coupled to the feedback terminal bulk3 of the third-stage first inverter (i.e., the feedback terminal of an odd number-stage first inverter).

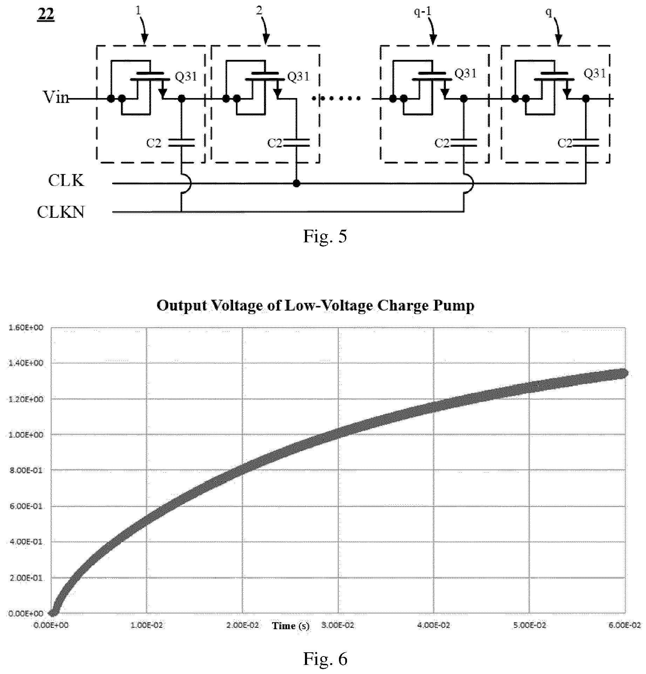

Fig. 5 is a circuit diagram of the low-voltage charge pump unit 22 in this embodiment. As shown in Fig. 5, the low-voltage charge pump unit 22 includes q charge pump elements configured as charge pump stages, such as first, second, ..., (q-1)-th and q-th charge pump stages as shown in Fig. 5, where q≥2. The low-voltage charge pump unit 22 can operate at a low voltage. That is, the low-voltage charge pump unit 22 can normally operate even when there is a small amount of charge remaining on the DC blocking capacitor C1.

With continued reference to Figs. 1a and 5, each of the charge pump elements includes a third NMOS transistor Q31 and a coupling capacitor C2. A gate, a drain and a substrate of the third NMOS transistor Q31 are connected and together serve as a power supply terminal of the charge pump element, and a source of the third NMOS transistor Q31 is coupled to one end of the coupling capacitor C2 and serves as an output terminal of the charge pump element. The other end of the coupling capacitor C2 serves as an input terminal of the charge pump element. Additionally, the output terminal of the s-th charge pump stage is coupled to the power supply terminal of the (s+1)-th charge pump stage, and the power supply terminal of the first-stage charge pump element serves as the power supply terminal of the low-voltage charge pump unit 22 for input of the voltage Vin. The output terminal of the q-th charge pump stage serves as the output terminal of the low-voltage charge pump unit 22 and is connected to the energy storage module 30, where 1≤s≤q-1. In this way, the q charge pump stages are connected in series.

With continued reference to Fig. 5, the input terminal of each odd-numbered charge pump stage serves as an input terminal of the low-voltage charge pump unit 22 for receiving the second clock signal CLKN, and the input terminal of each even-numbered charge pump stage serves as an input terminal of the low-voltage charge pump unit 22 for receiving the first clock signal CLK. For each even-numbered charge pump stage, when the first clock signal CLK is low level and the second clock signal CLKN is high level, a potential difference that is twice the amplitude of the clock signal is created between the drain and source of the third NMOS transistor Q31 by the coupling capacitor C2 of the specific stage. Meanwhile, since the substrate and drain of the third NMOS transistor Q31 are coupled together, the raised drain voltage results in an additionally reduced threshold voltage of the third NMOS transistor Q31, making the third NMOS transistor Q31 easier to turn on. On the contrary, when the first clock signal CLK is high level and the second clock signal CLKN is low level, as the gate and substrate of the third NMOS transistor Q31 are coupled to the low level, the third NMOS transistor Q31 will not be turned on, blocking reverse flow of charge and prevent reverse charging of the DC blocking capacitor C1 by the low-voltage charge pump unit 22, which may make energy recovery from the DC blocking capacitor C1 impossible.

Fig. 6 is a diagram showing simulation results of the energy recovery circuit in this embodiment. As shown in Fig. 6, assuming the energy storage module 30 is a capacitor of 20 pF with a voltage of 0.1 V remaining thereon, it can be charged to 1.27 V over 50 ms. Thus, the energy recovery circuit in this embodiment is capable of satisfactory energy recovery from the DC blocking capacitor C1.

It would be appreciated that although the time required by the energy recovery module 20 for energy recovery from the DC blocking capacitor C1 has been described above as 50 ms, the present invention is not so limited, because the energy recovery time can be flexibly adjusted by changing the number of stages (q) in the low-voltage charge pump unit 22.

### Embodiment 2

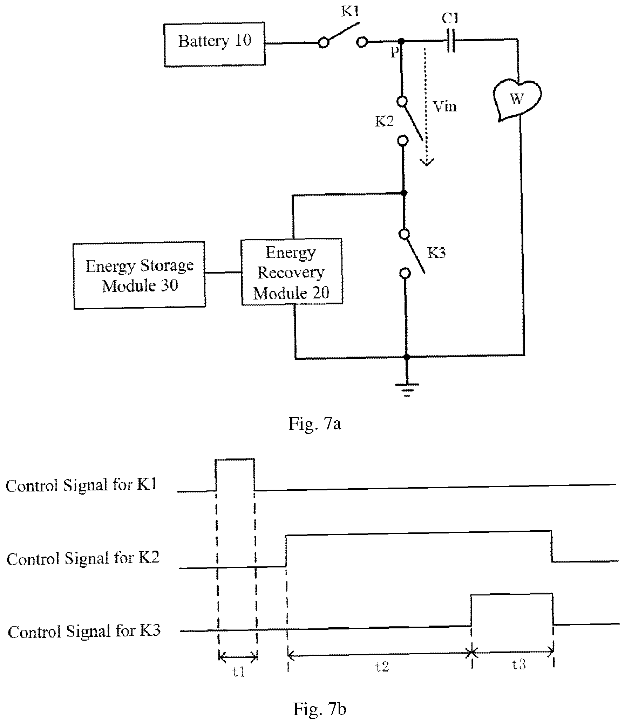

Fig. 7a shows a circuit diagram of a cardiac pacing system according to a second embodiment. As shown in Fig. 7a, this embodiment differs from the first embodiment in that the energy recovery circuit further includes a third on-off switching unit. Specifically, in this embodiment, the third on-off switching unit is a discharge switch K3. One end of the discharge switch K3 is coupled between the energy recovery module 20 and the energy recovery switch K2, and the other end thereof is connected to electrical ground.

It would be appreciated that the third on-off switching unit is not limited to being implemented as a switch, as it may be alternatively implemented as any other device or combination of devices capable of controlling the turn-on or turn-off of a circuit. For example, the third on-off switching unit may be alternatively implemented as a relay or a combination of multiple switches, but further description thereof is deemed unnecessary and is omitted.

Specifically, as shown in Fig. 7a, in this embodiment, the battery 10 is connected to one end of the pacing switch K1, and the other end of the pacing switch K1 is connected to the node P. The node P is connected to one end of the energy recovery switch K2, and the other end of the energy recovery switch K2 is connected to one end of the discharge switch K3 and the power supply terminal of the energy recovery module 20. The ground terminal of the energy recovery module 20 and the other end of the discharge switch K3 are connected to electrical ground. The node P is also connected to one end of the DC blocking capacitor C 1, and the other end of the DC blocking capacitor C1 is connected to one location of the myocardial tissue W. Another location of the myocardial tissue W is connected to electrical ground.

Fig. 7b illustrates control signals for the pacing switch K1, the energy recovery switch K2 and the discharge switch K3 in this embodiment. As shown in Fig. 7b, in a time interval t1, the pacing switch K1 is closed, and the energy recovery switch K2 and the discharge switch K3 are open. As a result, the energy recovery module 20 is turned off, and pacing pulses are output from the battery 10. After propagating through the DC blocking capacitor C1 which filters out a DC component thereof, the pacing pulses are transmitted to and stimulate the myocardial tissue W, causing the heart to beat and contract. With continued reference to Fig. 7b, in a time interval t2, the pacing switch K1 and the discharge switch K3 are open, and the energy recovery switch K2 is closed, turning off the battery 10. Charge on the DC blocking capacitor C1 flows into the energy recovery module 20 (manifested as a voltage Vin at the node P being applied to the energy recovery module 20). The energy recovery module 20 receives the charge and utilizes it to charge the energy storage module 30. As such, energy can be recovered by the energy recovery module 20 from the DC blocking capacitor C1 and stored in the energy storage module 30. With continued reference to Fig. 7b, in a time interval t3, the pacing switch K1 remains off, and the discharge switch K3 and the energy recovery switch K2 are both closed, causing release of charge remaining on the DC blocking capacitor C1 to electrical ground.

Compared with the first embodiment, in this embodiment, after the energy recovery module 20 recovers energy from the DC blocking capacitor C1 for a predetermined period time (the time interval t2), energy remaining on the DC blocking capacitor C1 is further released, thereby completely dissipating (recovering or releasing) energy in the DC blocking capacitor C1 and ensuring effective cardiac pacing in the next cycle. Further, as the function of releasing remaining charge on the DC blocking capacitor C1 to electrical ground is added, the predetermined period time required by the energy recovery module 20 to recover energy from the DC blocking capacitor C1 (time interval t2) needs not to be as long as is required by the previous embodiment.

### Embodiment 3

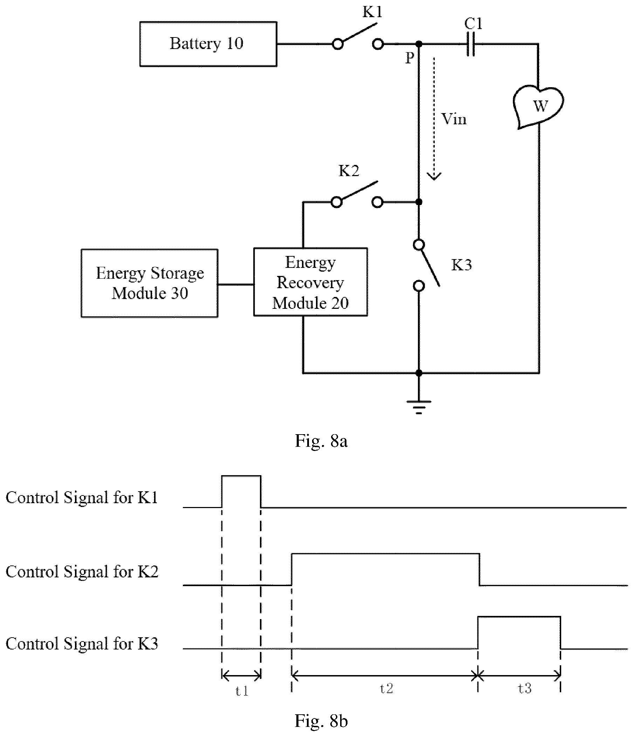

Fig. 8a shows a circuit diagram of a cardiac pacing system according to a third embodiment. As shown in Fig. 8a, this embodiment differs from the second embodiment in that, in this embodiment, one end of the discharge switch K3 is coupled between the node P and the energy recovery switch K2, the other end of the discharge switch K3 is connected to electrical ground.

Specifically, as shown in Fig. 8a, in this embodiment, the battery 10 is connected to one end of the pacing switch K1, and the other end of the pacing switch K1 is connected to the node P. The node P is connected to one end of the energy recovery switch K2 and one end of the discharge switch K3. The other end of the energy recovery switch K2 is connected to the power supply terminal of the energy recovery module 20, and the ground terminal of the energy recovery module 20 and the other end of the discharge switch K3 are connected to electrical ground. The node P is also connected to one end of the DC blocking capacitor C 1, and the other end of the DC blocking capacitor C1 is connected to one location of the myocardial tissue W. Another location of the myocardial tissue W is connected to electrical ground.

Fig. 8b illustrates control signals for the pacing switch K1, the energy recovery switch K2 and the discharge switch K3 in this embodiment. As shown in Fig. 8b, in a time interval t1, the pacing switch K1 is closed, and the energy recovery switch K2 and the discharge switch K3 are open. As a result, the energy recovery module 20 is turned off, and pacing pulses are output from the battery 10. After propagating through the DC blocking capacitor C1 which filters out a DC component thereof, the pacing pulses are transmitted to and stimulate the myocardial tissue W, causing the heart to beat and contract. With continued reference to Fig. 8b, in a time interval t2, the pacing switch K1 and the discharge switch K3 are open, and the energy recovery switch K2 is closed, turning off the battery 10. Charge on the DC blocking capacitor C1 flows into the energy recovery module 20 (manifested as a voltage Vin at the node P being applied to the energy recovery module 20). The energy recovery module 20 receives the charge and utilizes it to charge the energy storage module 30. As such, energy can be recovered by the energy recovery module 20 from the DC blocking capacitor C1 and stored in the energy storage module 30. With continued reference to Fig. 8b, in a time interval t3, the pacing switch K1 remains off, and the discharge switch K3 and the energy recovery switch K2 are both closed, causing release of charge remaining on the DC blocking capacitor C1 to electrical ground.

In summary, in the cardiac pacing systems provided in embodiments of the present invention, the second on-off switching unit of the energy recovery module is connected between the node between the first on-off switching unit and the DC blocking capacitor and electrical ground, and the energy storage module is connected to the energy recovery module. When the first on-off switching unit is closed and the second on-off switching unit is open, the battery can normally deliver pacing pulses to myocardial tissue. When the first on-off switching unit is open and the second on-off switching unit is closed, the energy recovery module recovers energy from the DC blocking capacitor and stores it in the energy storage module. The recovered energy can be used for subsequent pacing or normal operation of other modules in the cardiac pacing system, reducing a waste of energy while not adding additional power consumption. Such energy savings of the battery in the cardiac pacing system can extend a service life of the cardiac pacing system.

Presented above are merely a few preferred embodiments of the present invention, which do not limit the invention in any way. Changes in any forms made to the principles and teachings disclosed herein, including equivalents and modifications, by any person of ordinary skill in the art without departing from the scope of the invention are intended to fall within the scope of the invention.

## Claims

1. A cardiac pacing system, comprising a first on-off switching unit, a DC blocking capacitor and an energy recovery circuit, which are connected in sequence between a battery and electrical ground, the DC blocking capacitor connected to myocardial, the tissue myocardial tissue connected to electrical ground, the energy recovery circuit comprising an energy recovery module, a second on-off switching unit and an energy storage module,
wherein the energy recovery module is connected to the second on-off switching unit and then between a node between the first on-off switching unit and the DC blocking capacitor and electrical ground, and the energy storage module is connected to the energy recovery module; and
when the first on-off switching unit is closed and the second on-off switching unit is open, the battery delivers pacing pulses to the myocardial tissue, and when the first on-off switching unit is open and the second on-off switching unit is closed, the energy recovery module recovers energy from the DC blocking capacitor and stores the energy in the energy storage module.

2. The cardiac pacing system of claim 1, wherein the energy recovery module comprises a low-voltage oscillation unit and a low-voltage charge pump unit, wherein a power supply terminal of the low-voltage oscillation unit and a power supply terminal of the low-voltage charge pump unit are both configured to input voltage on the node; a ground terminal of the low-voltage oscillation unit and a ground terminal of the low-voltage charge pump unit are both connected to electrical ground;
an output terminal of the low-voltage oscillation unit is connected to an input terminal of the low-voltage charge pump unit; the low-voltage oscillation unit is configured to input two non-overlapping clock signals to the low-voltage charge pump unit; an output terminal of the low-voltage charge pump unit is connected to the energy storage module; and the low-voltage charge pump unit is configured to output a voltage to the energy storage module under the drive of the clock signals.

3. The cardiac pacing system of claim 2, wherein the low-voltage oscillation unit comprises a low-voltage oscillation circuit and a low-voltage buffer circuit, wherein a power supply terminal of the low-voltage oscillation circuit and a power supply terminal of the low-voltage buffer circuit together serve as the power supply terminal of the low-voltage oscillation unit, wherein a ground terminal of the low-voltage oscillation circuit and a ground terminal of the low-voltage buffer circuit together serve as the ground terminal of the low-voltage oscillation unit; and
an output terminal of the low-voltage oscillation circuit is connected to an input terminal of the low-voltage buffer circuit; the low-voltage oscillation circuit is configured to output two non-overlapping initial clock signals to the low-voltage buffer circuit; an output terminal of the low-voltage buffer circuit is connected to the input terminal of the low-voltage charge pump unit; and the low-voltage buffer circuit is configured to augment driving power of the two initial clock signals and then output the two clock signals to the low-voltage charge pump unit.

4. The cardiac pacing system of claim 3, wherein the low-voltage oscillation circuit comprises m stages first inverters, where m is an odd number greater than or equal to 3,
wherein power supply terminals of the m stages first inverters are connected and together serve as the power supply terminal of the low-voltage oscillation circuit; ground terminals of the m stages first inverters are connected and together serve as the ground terminal of the low-voltage oscillation circuit; an output terminal of an m-th stage first inverter is connected to an input terminal of a first-stage first inverter; an output terminal of an i-th stage first inverter is connected to an input terminal of an (i+1)-th stage o first inverter, where 1<i≤m-1; and
the two initial clock signals are output from the output terminals respectively of any odd number-stage of the first inverters and any even number-stage of the first inverters.

5. The cardiac pacing system of claim 4, wherein each of the first inverters comprises a first PMOS transistor and a first NMOS transistor,
the first PMOS transistor having a source serving as the power supply terminal of the first inverter, the first PMOS transistor having a gate connected to a gate of the first NMOS transistor and serving as the input terminal of the first inverter, the first PMOS transistor having a drain connected to a drain of the first NMOS transistor and serving as the output terminal of the first inverter, the first NMOS transistor having a source serving as the ground terminal of the first inverter, the first NMOS transistor having a substrate connected to a substrate of the first PMOS transistor and serving as a feedback terminal of the first inverter,
wherein the feedback terminal of the m-th stage first inverter is connected to the output terminal of the first-stage first inverter, and the feedback terminal of a j-th stage first inverter is connected to the output terminal of a (j+1)-th stage first inverter, where 1≤j≤m-1.

6. The cardiac pacing system of claim 5, wherein the low-voltage buffer circuit comprises two buffers, each buffer comprising n stages second inverters, where n > 2,
wherein power supply terminals of the n stage second inverters are connected and together serve as the power supply terminal of the low-voltage buffer circuit; ground terminals of the n stages second inverters are connected and together serve as the ground terminal of the low-voltage buffer circuit; an output terminal of an r-th stage second inverter is connected to an input terminal of an (r+1)-th stage second inverter; an input terminal of a first-stage second inverter and an output terminal of an n-th stage second inverter serve as an input terminal and an output terminal of the buffer, where 1≤r≤n-1; and
the two initial clock signals are input to the respective input terminals of the two buffers, and the clock signals are output from the respective output terminals of the two buffers.

7. The cardiac pacing system of claim 6, wherein each of the second inverters comprises a second PMOS transistor and a second NMOS transistor,
the second PMOS transistor having a source serving as the power supply terminal of the second inverter, the second PMOS transistor having a gate connected to a gate of the second NMOS transistor and serving as the input terminal of the second inverter, the second PMOS transistor having a drain connected to a drain of the second NMOS transistor and serving as the output terminal of the second inverter, the second NMOS transistor having a source serving as the ground terminal of the second inverter, the second NMOS transistor having a substrate connected to a substrate of the second PMOS transistor and serving as a feedback terminal of the second inverter,
wherein when the initial clock signal input to the buffer is output from an odd number-stage of the first inverters, the feedback terminal of each odd number-stage of the second inverters in the buffer is connected to the feedback terminal of any even number-stage of the first inverters, and the feedback terminal of each even number-stage of the second inverters in the buffer is connected to the feedback terminal of any odd number-stage of the first inverters; and
when the initial clock signal input to the buffer is output from an even number-stage of the first inverters, the feedback terminal of each odd number-stage of the second inverters in the buffer is connected to the feedback terminal of any odd number-stage of the first inverters, and the feedback terminal of each even number-stage of the second inverters in the buffer is connected to the feedback terminal of any even number-stage of the first inverters.

8. The cardiac pacing system of claim 2, wherein the low-voltage charge pump unit comprises q stages charge pump elements, where q≥2,
each of the charge pump elements comprising a third NMOS transistor and a coupling capacitor, the third NMOS transistor having a gate, a drain and a substrate, which are connected and together serve as a power supply terminal of the charge pump element, the third NMOS transistor having a source connected to one end of the coupling capacitor and serving as an output terminal of the charge pump element, the other end of the coupling capacitor serving as an input terminal of the charge pump element,
wherein the output terminal of an s-th stage of the charge pump elements is connected to the power supply terminal of an (s+1)-th stage of the charge pump elements, and the power supply terminal of a first stage of the charge pump elements and the output terminal of a q-th stage of the charge pump elements serve as the power supply terminal and the output terminal of the low-voltage charge pump unit, respectively, where 1≤s≤q-1; and
the input terminals of odd number-stages of the charge pump elements are connected and together serve as one input terminal of the low-voltage charge pump unit, the input terminal of each odd number-stage of the charge pump elements is configured for input of one of the clock signals, the input terminals of even number-stages of the charge pump elements are connected and together serve as another input terminal of the low-voltage charge pump unit, and the input terminal of each even number-stage of the charge pump elements is configured for input of the other one of the clock signals.

9. The cardiac pacing system of claim 1, wherein the energy recovery circuit further comprises a third on-off switching unit, one end of the third on-off switching unit is connected between the energy recovery module and the second on-off switching unit, and the other end of the third on-off switching unit is connected to electrical ground.

10. The cardiac pacing system of claim 1, wherein the energy recovery circuit further comprises a third on-off switching unit, one end of the third on-off switching unit is connected between the node and the second on-off switching unit, and the other end of the third on-off switching unit is connected to electrical ground.

11. The cardiac pacing system of claim 9 or 10, wherein during the delivery of the pacing pulses by the battery to the myocardial tissue, the recovery of energy by the energy recovery module from the DC blocking capacitor and the storage of the energy in the energy storage module, the third on-off switching unit is open.

12. The cardiac pacing system of claim 9, wherein after the energy recovery module recovers energy from the DC blocking capacitor for a predetermined period of time, both the third on-off switching unit and the second on-off switching unit are closed, thereby releasing remaining energy in the DC blocking capacitor.

13. The cardiac pacing system of claim 10, wherein after the energy recovery module recovers energy from the DC blocking capacitor for a predetermined period of time, the third on-off switching unit is closed and the second on-off switching unit is open, thereby releasing remaining energy in the DC blocking capacitor.
